# FASCICULE DE BREVET EUROPEEN

(11) **EP 0 604 802 B1**
(45) Date de publication et mention de la délivrance du brevet: **18.08.1999**
(21) Numéro de dépôt: 93119910.3
(22) Date de dépôt: 10.12.1993
(51) Int. Cl.: A23L 1/305, A23C 9/15, A23C 11/04

(54) **Composition alimentaire anti-cariogène**
Antikariogenisches Nahrungsmittel
Anticariogenic foodstuff

(30) Priorité: 28.12.1992 CH 397192
(43) Date de publication de la demande: 06.07.1994
(73) Titulaire: SOCIETE DES PRODUITS NESTLE S.A., 1800 Vevey (CH)
(72) Inventeur: Berrocal, Rafael, CH-1806 St-Legier (CH); Guggenheim, Bernhard, CH-8028 Zürich (CH); Neeser, Jean-Richard, CH-1073 Savigny (CH)
(74) Mandataire: Wavre, Claude-Alain

(56) Documents cités:
- EP-A- 0 316 938
- EP-A- 0 345 226
- WO-A-82/03008
- ARCHIVES OF ORAL BIOLOGY vol. 26 , 1981 pages 445 - 451 E. REYNOLDS ET AL. 'Effect of milk on caries incidence and bacterial composition of dental plaque in the rat'
- JOURNAL OF DAIRY SCIENCE. vol. 70, no. 2 , 1987 , CHAPAIGN, ILLINOIS US pages 392 - 396 C. WHITE 'Milk, milk products and dental health'
- JOURNAL OF NUTRITION vol. 55 , 1955 pages 399 - 414 G. SPERLING 'Effect of long feeding of whole milk diets to white rats'
- TECHNIQUE LAITIèRE no. 1028 , 1988 pages 21 - 23 J. FAUQUANT ET AL. 'Microfiltration du lait sur membrane minérale'

## Description

La présente invention a pour objet l'utilisation d'une caséine micellaire de lait écrémé dans la préparation d'une composition alimentaire anti-cariogène.

On connaît les propriétés anti-cariogènes de certains dérivés de la caséine.

C'est ainsi que US 5015628 (University of Melbourne) propose une composition anti-cariogène, notamment, comprenant comme agent actif des phosphopeptides obtenus par hydrolyse trypsique de caséine alpha ou béta, par exemple.

US 4994441 (Nestec S.A.) propose une composition anti-plaque et anti-carie dentaires dans laquelle l'agent actif est choisi parmi les kappa-caséino-glycopeptides et leurs dérivés désialylés.

Dans ce domaine également, FR 2672494 (Dr Madinier) propose des ferments prophylactiques favorisant la prévention de la carie dentaire, ces ferments pouvant être utilisés seuls, ou en association avec des ferments utilisés pour la production de yogourts, par exemple.

Archives of oral biology, vol 26 (1981), p 445-451 divulgue l'incidence possible du lait entier sur le mécanisme anticariogène. Le lait entier contient un certain nombre de composés, notamment les lipides, le lactose, des éléments traces, des immunoglobulines, des vitamines, des sels minéraux, des protéines, le citrate et des enzymes, qui ont une incidence sur le mécanisme anticariogène, par effet antibactérien sur la flore bucale ou par effet chimique sur la déminéralisation et la reminéralisation. Les résultats divulgués dans ce document (page 449, seconde colonne, second paragraphe) enseignent qu'il n'est pas possible de faire une association entre la composition bactérienne de la plaque dentaire et le fait d'administrer une composition laitière à des rats. Mais, par contre, qu'il est évident que l'incidence du lait entier sur le mécanisme anticariogène est du à une influence chimique directe sur le développement des caries, par effet de reminéralisation.

J. of Dairy Sci., vol 70, no 2 (1987), p 392-396 suppose que la caséine du lait peut réduire la solubilité de l'émail des dents par adsorption à leur surface. Les lipides contenus dans le lait pourraient de ce fait former un film protecteur à la surface des dents et, ainsi, prévenir une action antimicrobienne.

EP-A-345 226 enseigne l'utilisation de la caséine, obtenue par ultrafiltration, comme substitut de la graisse ou de la crème lors de la préparation de produits alimentaires crémeux dont le pH est supérieur à 5,6 et, en particulier, lors de la préparation de crémes glacées et de desserts congelés ou réfrigérés. De plus, ce document met en évidence, l'utilisation, de préférence, d'au moins 6% de caséine micellaire par rapport au poids total du produit alimentaire, de manière à conférer à ce dernier un goût onctueux.

La présente invention a pour but de proposer une utilisation d'une caseine micellaire de lait écrémé comme agent actif dans la préparation d'une composition alimentaire anticariogène.

On a constaté en effet avec surprise que la caséine elle-même présente une activité anti-cariogène remarquable, pour autant qu'elle se trouve sous sa forme micellaire.

La présente invention a également pour objet une utilisation d'une caséine micellaire de lait écrémé dans la préparation d'une composition alimentaire ayant une influence bénéfique sur la flore bucale.

Pour l'utilisation selon la présente invention, la caséine micellaire peut provenir d'un lait animal tel qu'un lait de vache, de chèvre ou de brebis, par exemple.

On peut obtenir cette caséine micellaire par microfiltration, ultrafiltration et/ou diafiltration dudit lait animal, par exemple.

De préférence, cette protéine micellaire est obtenue par microfiltration dudit lait animal sur une membrane minérale présentant une porosité de environ 0,1-0,2 µm, par exemple.

Dans une forme de réalisation préférée de l'utilisation selon la présente invention, on ajoute à ou l'on mélange avec ladite caséine micellaire, dans une proportion semblable à celle rencontrée dans un lait animal, des constituants dudit lait animal différents de la caséine.

Les exemples ci-après sont présentés à titre d'illustration de l'utilisation selon la présente invention, ainsi qu'à titre de démonstration des propriétés anti-cariogènes en question.

Dans ces exemples, les pourcentages et parties sont donnés en poids, sauf indication contraire.

### Exemple 1, exemples comparatifs i)-iii)

On prépare quatre compositions, l'une selon la présente invention, les trois autres pour comparaison, comprenant chacune 30% de saccharose, 32% de farine de blé, 5% de levure de bière, et 2% de micronutriments (mélange Gevral), les 30% restants étant composés de:
- exemple 1: poudre de lait de vache écrémé du commerce
- exemple comparatif i): substitut de poudre de lait de vache écrémé à base de caséinate composé lui-même de:

| | |
|---|---|
| caséinate de sodium | 41,9% |
| lactose | 49,1% |
| Ca₃(C₆H₅O₇)₂.4H₂O | 2,3% |
| CaCl₂ | 1,8% |
| MgCl₂.6H₂O | 0,7% |
| K₂HPO₄.3H₂O | 4,2% |

- exemple comparatif ii): substitut de poudre de lait de vache écrémé à base de lactoserum composé lui-même de:

| | |
|---|---|
| concentrat de protéines de lactoserum (80% de protéines) | 45,4% |
| lactose | 45,3% |
| Ca₃(C₆H₅O₇)₂.4H₂O | 2,0% |
| CaCl₂ | 1,5% |
| MgCl₂.6H₂O | 0,6% |
| K₂HPO₄.3H₂O | 3,4% |
| Na₂HPO₄ | 1,6% |
| histidine.HCl-H₂O | 0,2% |

- exemple comparatif iii): substitut de poudre de lait de vache écrémé à base de protéines de soya composé lui-même de :

| | |
|---|---|
| isolat de protéines de soya (91% de protéines) | 39,3% |
| lactose | 49,3% |
| Ca₃(C₆H₅O₇)₂.4H₂O | 2,4% |
| CaCl₂ | 1,9% |
| MgCl₂.6H₂O | 0,5% |
| K₂HPO₄.3H₂O | 4,3% |
| Na₂HPO₄ | 1,9% |
| L-méthionine | 0,3% |
| L-lysine.HCl | 0,1% |

On utilise dix portées de rats Osborne-Mendel à quatre rats par portée nés au jour 0. Au jour 13, on transfère les rats et leurs mères dans des cages en acier inoxydable à fond grillagé où on les nourrit avec des granulés de concentré Nafag et de l'eau du robinet à volonté jusqu'au jour 20. On répartit ensuite les rats au hasard par groupes de dix. On nourrit les rats de chaque groupe avec l'une des quatre compositions ci-dessus et on les abreuve avec de l'eau du robinet à volonté.

Aux jours 21 et 22, on administre à chaque rat, deux fois par jour, 100 µl de suspension concentrée de Streptococcus sobrinus (OMZ-176) et Actinomyces viscosus (Ny-1).

Au jour 40, on détermine l'importance de la plaque dentaire, le nombre de caries dentaires (fissures ébauchées, fissures avancées et caries de surface lisse) et le gain de poids, selon des procédures de routine. Les résultats sont présentés dans le tableau I ci-après.

Dans ce tableau, l'importance de la plaque dentaire est notée de 0 à 4, la note 0 équivalant à aucune plaque et la note 4 équivalant à une plaque d'extension maximale. Le nombre de fissures ébauchées et avancées est donné sur un maximum de 12. Le nombre de caries de surface lisse est donné sur un maximum de 20. Le gain de poids est donné en g.

**Tableau I**

| Ex No | Plaque dentaire | Fissures ébauchées | Fissures avancées | Caries de surf.lisse | Gain de poids |
|---|---|---|---|---|---|
| 1 | 2,8 | 7,1 | 2,7 | 6,2 | 105 |
| i) | 2,5 | 9,0 | 4,9 | 13,6 | 102 |
| ii) | 2,0 | 7,8 | 3,9 | 12,3 | 96 |
| iii) | 2,3 | 9,5 | 6,1 | 12,3 | 102 |

On voit que la meilleure diète est sans conteste celle où les rats consomment la composition selon l'exemple 1 qui comprend la caséine du lait de vache sous forme micellaire. Toutes les compositions essayées à titre de comparaison, dont aucune ne comprend de caséine micellaire, provoquent plus de caries alors que le gain de poids reste comparable.

### Exemples 2 et 3, exemples comparatifs iv) et v)

On prépare quatre compositions, deux selon la présente invention, les deux autres pour comparaison, comprenant chacune 30% de saccharose, 32% de farine de blé, 5% de levure de bière, et 2% de micronutriments (mélange Gevral), les 30% restants étant composés de:
- exemple 2: poudre de lait de vache écrémé du commerce
- exemple 3: lait de vache frais du commerce écrémé à 37-40°C et séché par atomisation à moins de 70°C
- exemple comparatif iv): lait de vache frais du commerce écrémé, acidifié à pH 5 par addition de HCl 2N, neutralisé par addition de KOH 2N et séché alors par atomisation
- exemple comparatif v): substitut de poudre de lait de vache écrémé à base de soya de même composition que celui décrit à l'exemple comparatif iii)

On utilise dix portées de rats Osborne-Mendel à quatre rats par portée nés au jour 0. Au jour 13, on transfère les rats et leurs mères dans des cages en acier inoxydable à fond grillagé où on les nourrit avec des granulés de concentré Nafag et de l'eau du robinet à volonté jusqu'au jour 20. On répartit ensuite les rats au hasard par groupes de dix. On nourrit les rats de chaque groupe avec l'une des quatre compositions ci-dessus et on les abreuve avec de l'eau du robinet à volonté.

Aux jours 21 et 22, on administre à chaque rat, deux fois par jour, 100 µl de suspension concentrée de Streptococcus sobrinus (OMZ-176) et Actinomyces viscosus (Ny-1).

Au jour 40, on détermine l'état microbiologique des rats. Pour ce faire, on prélève des échantillons par frotti buccal, on met les échantillons en suspension dans 5 ml de fluide RTF, et l'on pique des aliquots de 50 µl sur deux milieux de culture solide, à savoir des plaques d'agar au sang Columbia contenant 5% de sang humain hémolysé et des plaques d'agar TYS contenant 5% de saccharose.

Les plaques d'agar au sang sont utilisées pour déterminer le nombre total de bactéries anaérobies cultivables, autrement dit le nombre total de cfu ("colony forming units") anaérobies, ansi que le nombre de cfu de A.viscosus. Les plaques d'agar TYS sont utilisées pour déterminer le nombre total de bactéries cultivables sur ces plaques, ainsi que le nombre de cfu de S.sobrinus. Les résultats sont présentés dans le tableau II ci-après.

**Tableau II**

| Ex No | Agar au sang cfu x 10⁶ | | Agar TYS cfu x 10⁶ | |
|---|---|---|---|---|
| | total | A.viscosus | total | S.sobrinus |
| 2 | 78,0 | 45,8% | 63,4 | 15,0% |
| 3 | 55,0 | 39,6% | 40,8 | 11,1% |
| iv) | 62,0 | 18,5% | 44,6 | 45,9% |
| v) | 51,0 | 8,3% | 41,0 | 54,8% |

On voit sur ce tableau II que les diètes où les rats consomment les compositions selon les exemples 2 et 3, qui comprennent la caséine du lait de vache sous forme micellaire, ont une influence bénéfique sur la flore buccale puisqu'elles réduisent notablement la proportion de S.sobrinus mais qu'elles augmentent parallèlement la proportion d'A.viscosus qu'on y rencontre. On observe le phénomène contraire avec les diètes où les rats consomment les compositions selon les exemples comparatifs iv) et v), dont la première comprend une caséine dont la structure micellaire a été détruite et la seconde ne comprend pas de caséine.

Audit jour 40, on détermine également l'importance de la plaque dentaire, le nombre de caries dentaires (fissures ébauchées, fissures avancées et caries de surface lisse) et le gain de poids, selon des procédures de routine. Les résultats, chiffrés de manière analogue à ceux du Tableau I, sont présentés dans le tableau III ci-après.

**Tableau III**

| Ex No | Plaque dentaire | Fissures ébauchées | Fissures avancées | Caries de surf.lisse | Gain de poids |
|---|---|---|---|---|---|
| 2 | 1,6 | 9,3 | 4,5 | 4,8 | 116 |
| 3 | 1,3 | 8,8 | 3,9 | 4,3 | 111 |
| iv) | 1,6 | 9,9 | 5,8 | 7,3 | 112 |
| v) | 1,7 | 10,9 | 8,4 | 9,6 | 106 |

Les résultats présentés dans ce tableau III montrent que la diète où les rats consomment la composition selon l'exemple 3, qui comprend la caséine d'un lait de vache frais dont la forme micellaire n'a pas été détruite, est aussi bonne, voire même meilleure que la diète où les rats consomment la composition selon l'exemple 2 qui contient également la caséine du lait de vache sous forme micellaire. Par contre, la composition selon l'exemple comparatif iv) qui comprend la caséine d'un lait de vache frais dont la forme micellaire a été détruite, et la composition selon l'exemple comparatif v) qui ne comprend pas de caséine provoquent plus de caries alors que le gain de poids reste comparable.

### Exemples 4 et 5, exemple comparatif vi)

On prépare trois compositions, deux selon la présente invention, la troisième pour comparaison, comprenant chacune 40% de saccharose, 22% de farine de blé, 5% de levure de bière, et 2% de micronutriments (mélange Gevral), les 30% restants étant composés de:
- exemple 4: poudre de lait de vache écrémé du commerce
- exemple 5: substitut de poudre de lait écrémé composé lui-même de:

| | |
|---|---|
| caséine micellaire | 44,4% |
| lactose | 49,2% |
| Na₃C₆H₅O₇.2H₂O | 2,4% |
| KC1 | 1,1% |
| MgCl₂.6H₂O | 0,6% |
| K₂HPO₄.3H₂O | 2,3% |

la caséine micellaire a été préparée par microfiltration d'un lait écrémé de vache frais du commerce et séchage par atomisation
- exemple comparatif vi): substitut de poudre de lait de vache écrémé à base de caséinate de même composition que celui décrit à l'exemple comparatif i)

On utilise dix portées de rats Osborne-Mendel à trois rats par portée nés au jour 0. Au jour 13, on transfère les rats et leurs mères dans des cages en acier inoxydable à fond grillagé où on les nourrit avec des granulés de concentré Nafag et de l'eau du robinet à volonté jusqu'au jour 20. On répartit ensuite les rats au hasard par groupes de dix. On nourrit les rats de chaque groupe avec l'une des trois compositions ci-dessus et on les abreuve avec de l'eau du robinet à volonté.

Aux jours 21 et 22, on administre à chaque rat, deux fois par jour, 100 µl de suspension concentrée de Streptococcus sobrinus (OMZ-176) et Actinomyceo viscosus (Ny-1).

Au jour 40, on détermine l'état microbiologique des rats. Pour ce faire, on prépare des échantillons de plaque dentaire en broyant les première et seconde molaires de la mâchoire supérieure gauche de 6 rats de chaque groupe. On procède ensuite de la manière décrite ci-dessus aux exemples 2, 3, iv) et v). Les résultats sont présentés dans le tableau IV ci-après.

**Tableau IV**

| Ex No | Agar au sang cfu x 10⁷ | | Agar TYS cfu x 10⁷ | |
|---|---|---|---|---|
| | total | A.viscosus | total | S.sobrinus |
| 4 | 4,3 | 31,8% | 5,1 | 59,4% |
| 5 | 3,9 | 29,2% | 5,3 | 60,1% |
| vi) | 5,7 | 12,0% | 4,9 | 84,1% |

On voit sur ce tableau IV, et ces résultats confirment ceux présentés au tableau II, que les diètes où les rats consomment les compositions selon les exemples 4 et 5, qui comprennent la caséine du lait de vache sous forme micellaire, ont une influence bénéfique sur la flore buccale puisqu'elles réduisent notablement la proportion de S.sobrinus mais qu'elles augmentent parallèlement la proportion d'A.viscosus qu'on y rencontre. On observe ici également le phénomène contraire avec la diète où les rats consomment la composition, selon l'exemple comparatif vi), qui comprend une caséine dont la structure micellaire a été détruite.

Audit jour 40, on détermine également l'importance de la plaque dentaire, le nombre de caries dentaires (fissures ébauchées, fissures avancées et caries de surface lisse) et le gain de poids, selon des procédures de routine. Les résultats, chiffrés de manière analogue à ceux du Tableau I, sont présentés dans le tableau V ci-après.

**Tableau V**

| Ex No | Plaque dentaire | Fissures ébauchées | Fissures avancées | Caries de surf.lisse | Gain de poids |
|---|---|---|---|---|---|
| 4 | 1,8 | 11,0 | 8,0 | 13,9 | 100 |
| 5 | 1,2 | 10,1 | 4,4 | 9,6 | 99 |
| vi) | 2,0 | 11,4 | 9,5 | 19,3 | 100 |

Les résultats présentés dans ce tableau V montrent que la composition selon l'exemple 5, qui comprend la caséine d'un lait de vache frais dont la forme micellaire a été retenue par microfiltration, présente des propriétés anti-cariogènes particulièrement nettes si on la compare aux effets de la composition selon l'exemple comparatif vi) qui comprend une caséine de lait de vache dont la forme micellaire a été détruite. Les résultats de l'exemple 4 sont également bons comparés à ceux de l'exemple vi), si l'on prend en considération le fait que la teneur en saccharose des compositions de ce groupe d'exemple a été portée à 40% alors qu'elle était de 30% dans les groupes d'exemples précédents.

### Exemple 6

On prépare une composition pour une formule infantile de premier âge en mélangeant les composants suivants dans les proportions indiquées ci-après:

| | |
|---|---|
| caséine micellaire | 6,3% |
| poudre de lactoserum déminéralisé (12,5% de protéines) | 68,6% |
| matière grasse du lait | 20,6% |
| huile de maïs | 4,4% |
| K₂HPO₄ | 0,1% |

la caséine micellaire a été préparée par microfiltration d'un lait écrémé de vache frais du commerce et séchage par atomisation.

### Exemple 7

On prépare une composition pour une formule infantile de suite en mélangeant les composants suivants dans les proportions indiquées ci-après:

| | |
|---|---|
| caséine micellaire | 12,6% |
| poudre de lactoserum déminéralisé (12,5% de protéines) | 22,9% |
| lactose | 34,9% |
| matière grasse du lait | 20,4% |
| huile de maïs | 4,6% |
| minéraux, micronutriments et eau | 4,6% |

la caséine micellaire a été préparée par microfiltration d'un lait écrémé de vache frais du commerce et séchage par atomisation.

### Exemple 8

On prépare une composition pour une boisson lactée en mélangeant entre 3 et 15 g de caséine micellaire, préparée par microfiltration d'un lait écrémé de vache frais du commerce et séchage par atomisation, et entre 5 et 20 g de lactose, pour une quantité d'eau de 70 à 90 g, la somme de ces composants devant donner 100 g.

### Exemple 9

On prépare une composition pour un substitut de crème à café en poudre en mélangeant les composants suivants dans les proportions indiquées ci-après:

| | |
|---|---|
| caséine micellaire | entre 2 et 31% |
| lactose | entre 31 et 60% |
| huile de coco partiellement hydrogénée | 33% |
| minéraux, arômes et eau | 5% |

la somme de ces composants devant donner 100%.

La caséine micellaire a été préparée par microfiltration d'un lait écrémé de vache frais du commerce et séchage par atomisation.

## Revendications

1. Utilisation d'une caséine micellaire de lait écrémé comme agent actif dans la préparation d'une composition alimentaire anticariogène.

2. Utilisation selon la revendication 1, d'une caséine micellaire de lait écrémé dans la préparation d'une composition alimentaire ayant une influence bénéfique sur la flore bucale.

3. Utilisation selon la revendication 1, d'une caséine micellaire provenant d'un lait animal, notamment d'un lait de vache, de chèvre ou de brebis.

4. Utilisation selon la revendication 1, d'une caséine micellaire obtenue par microfiltration d'un lait animal.

5. Utilisation selon la revendication 1, d'une caséine micellaire obtenue par ultrafiltration et/ou diafiltration d'un lait animal.

6. Utilisation selon l'une des revendications 1-5, dans laquelle la composition alimentaire comprend de la caséine micellaire dans une proportion semblable à celle rencontrée dans le lait.

## Claims

1. Use of a micellar casein from skimmed milk as active agent in the preparation of an anticariogenic food composition.

2. Use according to claim 1, of a micellar casein from skimmed milk in the preparation of a food composition having a beneficial influence on the buccal flora.

3. Use according to claim 1, of a micellar casein derived from animal milk, in particular cow's, goat's or ewe's milk.

4. Use according to claim 1, of a micellar casein obtained by microfiltration of an animal milk.

5. Use according to claim 1, of a micellar casein obtained by ultrafiltration and/or diafiltration of an animal milk.

6. Use according to one of claims 1-5, wherein the food composition includes micellar casein in a proportion similar to that encountered in milk.

## Patentansprüche

1. Verwendung eines micellären Caseins aus Magermilch als Wirkstoff bei der Herstellung einer antikariogenen Nahrungsmittelzusammensetzung.

2. Verwendung nach Anspruch 1 eines micellären Caseins aus Magermilch bei der Herstellung einer Nahrungsmittelzusammensetzung, die einen vorteilhaften Einfluß auf die Mundflora aufweist.

3. Verwendung nach Anspruch 1 eines micellären Caseins, das aus einer Tiermilch stammt, insbesondere Kuhmilch, Ziegenmilch oder Schafmilch.

4. Verwendung nach Anspruch 1 eines micellären Caseins, das durch Mikrofiltration einer Tiermilch erhalten wurde.

5. Verwendung nach Anspruch 1 eines micellären Caseins, das durch Ultrafiltration und/oder Diafiltration einer Tiermilch erhalten wurde.

6. Verwendung nach einem der Ansprüche 1 bis 5, bei der die Nahrungsmittelzusammensetzung das micelläre Casein in einem Anteil enthält, der dem ähnlich ist, der in Milch gefunden wird.
